# EUROPEAN PATENT APPLICATION

(11) **EP 4 484 561 A1**
(43) Date of publication of application: **01.01.2025**
(21) Application number: 23759079.9
(22) Date of filing: 16.02.2023
(51) Int. Cl.: C12N 15/86, A61K 48/00, A61P 27/02

(54) **EXPRESSION CASSETTE COMBINATION AND USE THEREOF**

(30) Priority: 25.02.2022 CN 202210181036
(71) Applicant: Chigenovo Co., Ltd., Beijing 102206 (CN)
(72) Inventor: ZHONG, Xiancheng, Beijing 102206 (CN); CHEN, Shaohong, Beijing 102206 (CN); SHI, Tianyong, Beijing 102206 (CN)
(74) Representative: Hoffmann Eitle
(86) International application number: PCT/CN2023/076459
(87) International publication number: WO 2023/160454

(57) **Abstract**

The present application relates to expression cassette combinations and uses thereof. The expression cassette combination comprises a first expression cassette and a second expression cassette, wherein the first expression cassette can express an N-terminal truncated version of an ABCA4 protein, the second expression cassette can express a C-terminal truncated version of an ABCA4 protein, and the N-terminal truncated version of the ABCA4 protein and the C-terminal truncated version of the ABCA4 protein can possess the biological functions of a complete ABCA4 protein without forming the complete ABCA4 protein therefrom.

## Description

### TECHNICAL FIELD

The present application relates to the field of biomedicine, in particular to expression cassette combinations and uses thereof.

### BACKGROUND

ABCA4 is a lipid inward flippase in retinal photoreceptor cells or retinal pigment epithelial cells, which can transport retinylphosphatidylethanolamine (NRPE) (*i.e.*, the lipid derivative formed by phosphatidylethanolamine (PE) and all-trans retinaldehyde (ATR)) from the intra-cavity side of the outer segment membranous disc to the cytoplasm side. Subsequently, NRPE will be hydrolyzed into ATR and PE, and ATR will be catalyzed by retinal reductase in the cytoplasm to form all-trans retinol (ATRol), thereby returning to the visual cycle. If the lipid transport function of ABCA4 is impaired, ATR and NRPE will accumulate excessively on the intra-cavity side of the outer segment membranous disc. The excessive accumulations of ATR and NRPE will further irreversibly form toxic di-retinyl derivatives, eventually causing a series of retinal degenerative diseases, including the most frequently hereditary macular degeneration, age-related macular degeneration, retinitis pigmentosa, cone-rod dystrophy, and the like.

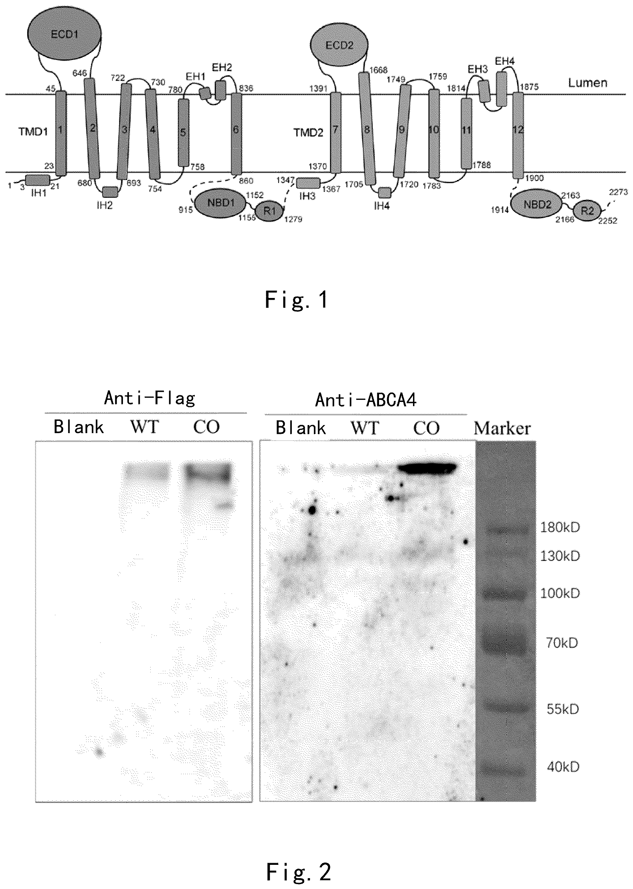

The human ABCA4 CDS is 6.7kb in full length and encodes 2,273 amino acids. The schematic diagram of the structure of ABCA4 is shown in FIG. 1 (Xie, T., Z. Zhang, Q. Fang, B. Du, and X. Gong (2021). "Structural basis of substrate recognition and translocation by human ABCA4." Nat Commun 12(1): 3853).

At present, there are no marketed drugs for retinal diseases caused by ABCA4 mutations.

### SUMMARY

The present application provides an expression cassette combination. The expression cassette combination can co-express an N-terminal truncated version (an N-terminal domain) of an ABCA4 protein and a C-terminal truncated version (a C-terminal domain) of an ABCA4 protein expressed by a second expression cassette in a cell. The N-terminal truncated version of the ABCA4 protein and the C-terminal truncated version of the ABCA4 protein can possess the biological functions of a complete ABCA4 protein (*e.g.*, cellular localization consistent with the complete ABCA4 protein; ATP hydrolase activity; and/or co-expression in photoreceptor cells, reducing retinal A2E deposition), without forming the complete ABCA4 protein therefrom.

The present application creatively discovered that, as the N-terminal truncated version of the ABCA4 protein and the C-terminal truncated version of the ABCA4 protein can possess the biological functions of the complete ABCA4 protein without forming the complete ABCA4 protein therefrom, there is no need to transfect and express the complete ABCA4 protein to achieve the therapeutic purpose. At present, a dual-AAV system is commonly used for the transfection and expression of the complete ABCA4 protein. For example, the complete ABCA4 expression frame is split into two parts, and these two parts share a set of promoter, stop codon, poly A, etc. required for expression. DNA homologous recombination or RNA splicing in the cell is used to obtain a complete RNA, which ultimately encodes the complete ABCA4 protein; or protein splicing is used to form the complete ABCA4 protein. By adopting a DNA homologous recombination method, dual AAV vectors were injected into the subretinal space of mice, each AAV was injected at 1×10¹⁰ vg/eye, and the expression intensity of the ABCA4 protein was about 1% of the endogenous expression (McClements, M. E., et al. (2019). "An AAV Dual Vector Strategy Ameliorates the Stargardt Phenotype in Adult Abca4(-/-) Mice." Hum Gene Ther 30(5): 590-600). Even by adopting a hybrid method that combines the homologous recombination and the RNA splicing, dual AAV vectors were injected into the subretinal space of mice, each AAV was injected at 3×10⁹ vg/eye, and the expression intensity of the ABCA4 in mice was about 10% of the endogenous expression (Dyka, F. M., et al. (2019). "Dual ABCA4-AAV Vector Treatment Reduces Pathogenic Retinal A2E Accumulation in a Mouse Model of Autosomal Recessive Stargardt Disease." Hum Gene Ther 30(11): 1361-1370). By adopting an intein protein splicing method, dual AAV vectors were injected into the subretinal space of mice, each AAV was injected at 5×10⁹ vg/eye, and the expression intensity of the ABCA4 in mice was still no higher than 10% of the endogenous expression (Tomabene, P., et al. (2019). "Intein-mediated protein trans-splicing expands adeno-associated virus transfer capacity in the retina." Sci Transl Med 11(492)). As the above methods have low expression efficiencies, a large number of AAV viruses need to be injected during the treatment in order to reach or approach the endogenous expression level, and thus the immunotoxicity will be too high. However, these challenges are solved by the expression cassette combination provided in the present application.

In one aspect, the present application provides an expression cassette combination, comprising a first expression cassette and a second expression cassette, wherein the first expression cassette can express an N-terminal truncated version of an ABCA4 protein, the second expression cassette can express a C-terminal truncated version of an ABCA4 protein, and the N-terminal truncated version of the ABCA4 protein expressed by the first expression cassette and the C-terminal truncated version of the ABCA4 protein expressed by the second expression cassette can possess the biological functions of a complete ABCA4 protein without forming the complete ABCA4 protein therefrom.

In certain embodiments, the N-terminal truncated version of the ABCA4 protein expressed by the first expression cassette and the C-terminal truncated version of the ABCA4 protein expressed by the second expression cassette do not substantially interact to form the complete ABCA4 protein.

In certain embodiments, the protein molar ratio of the N-terminal truncated version of the ABCA4 protein expressed by the first expression cassette to the C-terminal truncated version of the ABCA4 protein expressed by the second expression cassette is about 3:1 to 1:3.

In certain embodiments, the first expression cassette and/or the second expression cassette do not express a junction sequence enabling the N-terminal truncated version of the ABCA4 protein expressed by the first expression cassette and the C-terminal truncated version of the ABCA4 protein expressed by the second expression cassette to form the complete ABCA4 protein.

In certain embodiments, the junction sequence enables the N-terminal truncated version of the ABCA4 protein expressed by the first expression cassette and the C-terminal truncated version of the ABCA4 protein expressed by the second expression cassette to form the complete ABCA4 protein through a method selected from the group consisting of homologous recombination, mRNA splicing, and protein splicing.

In certain embodiments, the junction sequence comprises a homologous arm sequence.

In certain embodiments, the junction sequence comprises a sequence capable of being complementary to a sequence encoding at least a portion of the N-terminal truncated version of the ABCA4 protein; and/or the junction sequence comprises a sequence capable of being complementary to a sequence encoding at least a portion of the C-terminal truncated version of the ABCA4 protein.

In certain embodiments, the first expression cassette does not comprise a sequence capable of being complementary to a sequence encoding at least a portion of the N-terminal truncated version of the ABCA4 protein; and/or the second expression cassette does not comprise a sequence capable of being complementary to a sequence encoding at least a portion of the C-terminal truncated version of the ABCA4 protein.

In certain embodiments, the junction sequence comprises a splicing signal of mRNA intron.

In certain embodiments, the first expression cassette does not comprise an intron upstream splicing acceptor site (SA); and/or the second expression cassette does not comprise an intron downstream splicing donor site (SD).

In certain embodiments, the junction sequence comprises an intein.

In certain embodiments, the junction sequence comprises a sequence encoding an intein protein.

In certain embodiments, the first expression cassette does not express a sequence encoding the N-terminal portion of the intein protein; and/or the second expression cassette does not express a sequence encoding the C-terminal portion of the intein protein.

In certain embodiments, the N-terminal truncated version of the ABCA4 protein comprises N-terminal domain of the ABCA4 protein: transmembrane domains TMD1 to TMD6, extracellular domains ECD1, IH1, IH2, EH1, EH2, NBD1, and/or R1; preferably transmembrane domains TMD1 to TMD6, extracellular domains ECD1, IH2, EH1, EH2, and NBD1, and optionally IH1 and/or R1. In particularly preferred embodiments, the N-terminal truncated version of the ABCA4 protein comprises amino acids 1 to 1160 (aa 1-1160) (SEQ ID NO: 35), amino acids 1 to 1220 (aa 1-1220) (SEQ ID NO: 36), amino acids 1 to 1280 (aa 1-1280) (SEQ ID NO: 37), amino acids 1 to 1347 (aa 1-1347) (SEQ ID NO: 2), or amino acids 1 to 1280 and 2253-2273 (aa 1-1280*, 2253-2273) (SEQ ID NO: 38) at the N-terminal of the ABCA4 protein. In certain embodiments, the N-terminal truncated version of the ABCA4 protein may not comprise IH1 and/or R1.

In certain embodiments, the N-terminal truncated version of the ABCA4 protein comprises, sequentially from the N-terminus, IH1, TMD1, ECD1, TMD2, IH2, TMD3, TMD4, TMD5, EH1, EH2, TMD6, NBD1, and/or R1.

In certain embodiments, the N-terminal truncated version of the ABCA4 protein comprises an amino acid sequence set forth in SEQ ID NO: 2 or 4.

In certain embodiments, the first expression cassette comprises a nucleotide sequence encoding the N-terminal truncated version of the ABCA4 protein.

In certain embodiments, the nucleotide sequence encoding the N-terminal truncated version of the ABCA4 protein is set forth in SEQ ID NO: 25 or 27.

In certain embodiments, the first expression cassette comprises a promoter.

In certain embodiments, the C-terminal truncated version of the ABCA4 protein comprises C-terminal domains of the ABCA4 protein: transmembrane domains TMD7 to TMD12, extracellular domains ECD2, IH3, IH4, EH3, EH4, NBD2, and/or R2; preferably transmembrane domains TMD7 to TMD12, extracellular domains ECD2, IH4, EH3, EH4, and NBD2, and optionally IH3 and/or R2. In particularly preferred embodiments, the C-terminal truncated version of the ABCA4 protein comprises amino acids 1348-2273 (aa 1348-2273), amino acids 1369-2273 (aa 1369-2273), amino acids 1348-2170 (aa 1348-2170), or amino acids 1-20 and 1369-2273 (aa 1-20, 1369-2273) at the C-terminal of the ABCA4 protein. In certain embodiments, the C-terminal truncated version of the ABCA4 protein may not comprise IH3 and/or R2.

Without being limited to any theory, it is believed that the IH1/3 domain of the ABCA4 protein provides a membrane localization signal, and the R1/2 domain provides a portion of the interaction in the intracellular region, but these two domains do not seem to be necessary for the integrity of the N+C structure and function.

In certain embodiments, the C-terminal truncated version of the ABCA4 protein comprises, sequentially from the N-terminus, IH3, TMD7, ECD2, TMD8, IH4, TMD9, TMD10, TMD11, EH3, EH4, TMD12, NBD2, and/or R2.

In certain embodiments, the C-terminal truncated version of the ABCA4 protein comprises an amino acid sequence set forth in SEQ ID NO: 3 or 5.

In certain embodiments, the second expression cassette comprises a nucleotide sequence encoding the C-terminal truncated version of the ABCA4 protein.

In certain embodiments, the nucleotide sequence encoding the C-terminal truncated version of the ABCA4 protein is set forth in SEQ ID NO: 26 or 28.

In certain embodiments, the second expression cassette comprises a promoter.

In certain embodiments, the first expression cassette expresses a first constant region, and/or the second expression cassette expresses a second constant region, wherein the first constant region and the second constant region can interact such that the N-terminal truncated version of the ABCA4 protein expressed by the first expression cassette and the C-terminal truncated version of the ABCA4 protein expressed by the second expression cassette interact to form an N-C heterodimer of the ABCA4 protein.

In certain embodiments, the first constant region has a first modification, and/or the second constant region has a second modification, wherein the first modification and the second modification can promote the formation of the heterodimer.

In certain embodiments, the first constant region and the second constant region are derived from a constant region of an antibody.

In certain embodiments, the first modification and the second modification comprise a knob-into-hole modification.

In certain embodiments, the first constant region comprises an amino acid sequence set forth in SEQ ID NO: 6 or 7.

In certain embodiments, the second constant region comprises an amino acid sequence set forth in SEQ ID NO: 7 or 6.

In certain embodiments, the first expression cassette comprises, from the 5' end, a promoter sequence, a nucleotide sequence encoding the N-terminal truncated version of the ABCA4 protein, and/or a nucleotide sequence encoding the first constant region.

In certain embodiments, the first expression cassette is consisted of, from the 5' end, a promoter sequence and a nucleotide sequence encoding the N-terminal truncated version of the ABCA4 protein.

In certain embodiments, the first expression cassette is consisted of, from the 5' end, a nucleotide sequence of a promoter, a nucleotide sequence encoding the first constant region, and a nucleotide sequence encoding the N-terminal truncated version of the ABCA4 protein.

In certain embodiments, the first expression cassette is consisted of, from the 5' end, a nucleotide sequence of a promoter, a nucleotide sequence encoding the N-terminal truncated version of the ABCA4 protein, and a nucleotide sequence encoding the first constant region.

In certain embodiments, the second expression cassette comprises, from the 5' end, a promoter sequence, a nucleotide sequence encoding the C-terminal truncated version of the ABCA4 protein, and/or a nucleotide sequence encoding the second constant region.

In certain embodiments, the second expression cassette is consisted of, from the 5' end, a promoter sequence and a nucleotide sequence encoding the C-terminal truncated version of the ABCA4 protein.

In certain embodiments, the second expression cassette is consisted of, from the 5' end, a nucleotide sequence of a promoter, a nucleotide sequence encoding the C-terminal truncated version of the ABCA4 protein, and a nucleotide sequence encoding the second constant region.

In certain embodiments, the second expression cassette is consisted of, from the 5' end, a promoter sequence, a nucleotide sequence encoding the second constant region, and a nucleotide sequence encoding the C-terminal truncated version of the ABCA4 protein.

In certain embodiments, the first expression cassette comprises a nucleotide sequence set forth in any of SEQ ID NOs: 2, 4, 8-10, 15-18, and 34.

In certain embodiments, the second expression cassette comprises a nucleotide sequence set forth in any of SEQ ID NOs: 3, 5, 11-14, and 19-22.

In certain embodiments, the first expression cassette and the second expression cassette are present individually.

In certain embodiments, the first expression cassette and/or the second expression cassette are present in the form selected from the group consisting of: an isolated nucleic acid molecule, a liposome, and/or an exosome.

In certain embodiments, the first expression cassette and/or the second expression cassette are present in the form of a plasmid.

In certain embodiments, the first expression cassette and/or the second expression cassette are viral vectors.

In certain embodiments, the first expression cassette and/or the second expression cassette are AAVs.

In certain embodiments, in the expression cassette combination, the molar ratio of the protein expressed by the first expression cassette to the protein expressed by the second expression cassette is about 1:3 to 3:1.

In certain embodiments, in the expression cassette combination, the molar ratio of the protein expressed by the first expression cassette to the protein expressed by the second expression cassette is about 1:1.

In another aspect, the present application provides a kit comprising the expression cassette combination described herein.

In certain embodiments, the kit comprises reagents and/or instruments for administrating the expression cassette combination.

In another aspect, the present application provides the use of the expression cassette combination described herein and/or the kit described herein in the manufacture of a medicament for treating a disease, wherein the disease comprises a disease caused by an ABCA4 mutation.

In certain embodiments, the disease comprises an inherited retinal disease.

In certain embodiments, the disease comprises a hereditary macular degeneration disease, age-related macular degeneration, retinitis pigmentosa, and/or cone-rod dystrophy.

In another aspect, the present application provides a method for expressing a heterologous ABCA4 gene, comprising administrating the expression cassette combination described herein and/or the kit described herein to a subject in need thereof.

In another aspect, the present application provides a method for alleviating the cell death caused by retinylphosphatidylethanolamine (NRPE), comprising administrating the expression cassette combination described herein and/or the kit described herein to a subject in need thereof.

In another aspect, the present application provides a method for treating a disease caused by an ABCA4 mutation, comprising administrating the expression cassette combination described herein and/or the kit described herein to a subject in need thereof.

In certain embodiments, the administrating comprises injection.

In certain embodiments, the disease comprises an inherited retinal disease.

In certain embodiments, the disease comprises a hereditary macular degeneration disease, age-related macular degeneration, retinitis pigmentosa, and/or cone-rod dystrophy.

Other aspects and advantages of the present application can be readily appreciated by those skilled in the art from the detailed descriptions below. Only exemplary embodiments of the present application are shown and described in the detailed descriptions below. As will be recognized by those skilled in the art, the content of the present application will enable those skilled in the art to make changes to the particular embodiments without departing from the spirit and scope of the invention disclosed in the present application. Accordingly, the accompanying drawings and the descriptions in the specification of the present application are only exemplary and not restrictive.

### DESCRIPTION OF THE DRAWINGS

The specific features of the invention disclosed in the present application are set forth in the appended claims. The characteristics and advantages of the invention disclosed in the present application can be better understood by reference to the exemplary embodiments described in detail below and the accompanying drawings. Brief descriptions of the accompanying drawings are as follows:
FIG. 1 shows a schematic diagram of the structure of ABCA4.
FIG. 2 shows the protein expression intensity of the codon-optimized ABCA4.
FIG. 3 shows the dimerization of the N-terminal truncated version (aa 1-1347) of the ABCA4 protein and the C-terminal truncated version (aa 1348-2273) of the ABCA4 protein described in the present application.
FIG. 4 shows that there is an interaction between the N-terminal truncated version (aa 1-1347) of the ABCA4 protein and the C-terminal truncated version (aa 1348-2273) of the ABCA4 protein described in the present application.
FIG. 5 shows that there is an interaction between the N-terminal truncated versions of the ABCA4 protein and the C-terminal truncated versions of the ABCA4 protein with different lengths or containing different domains.
FIG. 6 shows that there is a cell co-localization between the N-terminal truncated version (aa 1-1347) of the ABCA4 protein and the C-terminal truncated version (aa 1348-2273) of the ABCA4 protein described in the present application.
FIG. 7 shows that the co-localization pattern of the N-terminal truncated version (aa 1-1347) of the ABCA4 protein and the C-terminal truncated version (aa 1348-2273) of the ABCA4 protein described in the present application is consistent with that of the full-length wild-type ABCA4 protein.
FIG. 8 shows the ATP hydrolase activity by the transfection of the N-terminal truncated version (aa 1-1347) of the ABCA4 protein and/or the C-terminal truncated version (aa 1348-2273) of the ABCA4 protein described in the present application.
FIG. 9 shows that there is an interaction between the N-terminal truncated version (aa 1-1347) of the ABCA4 protein and the C-terminal truncated version (aa 1348-2273) of the ABCA4 protein with modifications described in the present application.
FIG. 10 shows that there is an interaction between the N-terminal truncated versions-Fca of the ABCA4 protein and the Fcb-C-terminal truncated versions or the C-terminal truncated versions-Fcb of the ABCA4 protein with different lengths or containing different domains.
FIG. 11 shows the ATP hydrolase activity by the transfection of the N-terminal truncated version (aa 1-1347) of the ABCA4 protein and/or the C-terminal truncated version (aa 1348-2273) of the ABCA4 protein with modifications described in the present application .
FIG. 12 shows the alleviation of cell death caused by NRPE by the transfection of the N-terminal truncated version (aa 1-1347) of the ABCA4 protein and/or the ABCA4 protein described in the present application (with modifications).
FIG. 13 shows that the co-infection of the complex of AAV-ABCA4 N-terminal truncated version of and AAV-ABCA4 C-terminal truncated version can alleviate the cell death caused by NRPE.

### DESCRIPTION OF EMBODIMENTS

The embodiments of the invention in the present application are described below by certain specific Examples, and those skilled in the art can easily understand other advantages and effects of the invention in the present application from the disclosure of this specification.

### Definitions of terms

The term "first expression cassette" generally refers to an exogenous DNA sequence that is operably linked to a promoter or other regulatory sequences sufficient to direct the transcription of a target gene. As used herein, the expression cassette generally refers to a nucleic acid construct that, when introduced into a host cell, causes the transcription and/or translation of an RNA or polypeptide respectively. The expression cassette may have a series of specialized nucleic acid elements that allow the transcription of a specific nucleic acid in a target cell. The expression cassette may be incorporated into a plasmid, chromosome, mitochondrial DNA, plasmid DNA, virus, or nucleic acid fragment. The expression cassette may comprise an untranslated or untranslatable antisense or sense construct. In the cases of expressing a transgene and suppressing an endogenous gene (*e.g.*, by antisense, RNAi or sense suppression), those skilled in the art will recognize that the inserted polynucleotide sequence does not need to be identical, while may only be substantially identical to the sequence from which it is derived. The expression cassette may comprise a polynucleotide construct of a transcription factor operably linked to a promoter, which may be a promoter obtained from a gene regulated by the transcription factor. As used herein, the first expression cassette may express a protein (*e.g.*, the N-terminal truncated version of the ABCA4 protein).

The term "second expression cassette" generally refers to an expression cassette that is not completely identical to the first expression cassette. For example, the second expression cassette may express a protein different from the protein expressed by the first expression cassette (*e.g.*, the C-terminal truncated version of the ABCA4 protein).

The term "expression cassette combination" generally refers to a combination comprising at least one expression cassette. For example, the expression cassette combination described herein may comprise the first expression cassette and the second expression cassette.

The term "ABCA4 gene" generally refers to a gene encoding the rim protein or RmP. The ABCA4 gene is also known as an ABCR gene. This gene was first cloned and identified as a gene that may cause the Stargardt disease, which is an autosomal recessive disease and may lead to macular degeneration. The NCBI Entrez Gene accession number for human ABCA4 gene is 24.

The term "complete ABCA4 protein" generally refers to ATP-binding cassette subfamily A, Member 4, with a complete structure. The complete ABCA4 protein may be encoded by human ABCA4 gene. ABCA4 is a lipid inward flippase in retinal photoreceptor cells or retinal pigment epithelial cells, which can transport retinylphosphatidylethanolamine (NRPE) (*i.e.*, the lipid derivative formed by phosphatidylethanolamine (PE) and all-trans retinaldehyde (ATR)) from the intra-cavity side of the outer segment membranous disc to the cytoplasm side. Subsequently, NRPE will be hydrolyzed into ATR and PE, and ATR will be catalyzed by retinal reductase in the cytoplasm to form all-trans retinol (ATRol), thereby returning to the visual cycle. The UniProt accession number for the complete human ABCA4 protein is P78363.

The term "N-terminal truncated version of the ABCA4 protein" generally refers to an N-terminal truncated version of the complete human ABCA4 protein. For example, the N-terminal truncated version of the ABCA4 protein may comprise an amino acid sequence set forth in amino acids 1-1325, amino acids 1-1160, amino acids 1-1220, amino acids 1-1280, or amino acids 1-1347 from the N-terminus of the complete human ABCA4 protein. For example, the amino acid sequence of the N-terminal truncated version of the ABCA4 protein may be as set forth in amino acids 1-1325 from the N-terminus of the complete human ABCA4 protein. As used herein, the N-terminal truncated version of the ABCA4 protein and/or the C-terminal truncated version of the ABCA4 protein may correspond to the blue and green structures in Figure 1 of Nature Communications volume 12, Article number: 3853 (2021), respectively.

The term "C-terminal truncated version of the ABCA4 protein" generally refers to a C-terminal truncated version of the complete human ABCA4 protein. For example, the C-terminal truncated version of the ABCA4 protein may comprise an amino acid sequence set forth in amino acids 1326-2273, amino acids 1348-2273, amino acids 1369-2273, or amino acids 1348-2170 from the N-terminus of the complete human ABCA4 protein. For example, the amino acid sequence of the C-terminal truncated version of the ABCA4 protein may be as set forth in amino acids 1326-2273 from the N-terminus of the complete human ABCA4 protein.

The term "biological function" generally refers to an activity that is native to or is a desired activity of the tested biological entity, for example the natural activity of a cell, protein, or the like. Ideally, the presence of a biological function may be tested by an *in vitro* functional assay.

The term "homologous recombination" generally means that the recombination is occurred as a result of the interaction between homologous or identical fragments of genetic materials. For example, the homologous recombination may comprise the exchange of DNA fragments between two DNA molecules. The exchanged fragments may be fragments flanking to the sites where the two DNA molecules have identical nucleic acid sequences (i.e., "homologous regions"). The "homologous regions" may comprise nucleic acid sequence ranges that are homologous to each other on the nucleic acid fragments involved in the homologous recombination. These homologous regions may have a length of at least about 10 bp.

The term "mRNA splicing" generally refers to a cellular event that is occurred in the nucleus of a eukaryotic cell, in which an intron is removed from a pre-mRNA type. Generally, this process may require the formation of a spliceosome complex, in which a 5' splicing donor site is brought into the proximity to a 3' splicing acceptor site, while an interfering intron sequence is removed from the transcript.

The term "protein splicing" generally refers to a process in which an internal region (intein) of a precursor protein is cleaved and the flanking regions (exteins) of the protein are joined to form a mature protein. The intein unit may comprise the necessary components required to catalyze the protein splicing, and generally comprises an endonuclease domain involved in the mobility of the intein. The resulting proteins may be linked together, but not expressed as an individual protein. The protein splicing may also spontaneously combine with split inteins expressed on separate polypeptides to form a single intein, which then undergoes the protein splicing process to combine into separate proteins, thereby occurring in a trans way.

The term "homologous arm sequence" generally refers to a sequence having sufficient homology to a corresponding target sequence within a cell genome to undergo homologous recombination. For example, the homologous arm sequence may comprise a polynucleotide sequence having at least 80%, at least 85%, at least 90%, at least 91%, at least 92%, at least 93%, at least 94%, at least 95%, at least 96%, at least 97%, at least 98%, at least 99%, or 100% identity to the target sequence. The homologous arm sequence may comprise an upstream homologous arm sequence and a downstream homologous arm sequence.

The term "splicing signal of mRNA intron" generally refers to a signal for RNA splicing. For example, the RNA splicing may comprise intramolecular splicing (cis splicing) and intermolecular splicing (trans splicing). In an intron, a 5' splicing site (splice site), a 3' splicing site, and a splicing branch point may be required for splicing. The splicing may be catalyzed by a spliceosome, which is constituted by a large RNA-protein complex (called as small nuclear ribonucleoprotein (snRNP)) composed of five different small nuclear RNAs (snRNAs) and no less than one hundred proteins. The RNA of the small nuclear ribonucleoprotein will undergo the hybridization with the intron and be involved in the catalytic reaction of splicing.

The term "upstream splicing acceptor site (SA)" generally refers to a splicing acceptor (splice acceptor, SA). In certain cases, there are regions (called as SD and SA) at the junctions of the upstream and the downstream of one intron with the adjacent exons respectively. On the early mRNA chain formed after transcription, the sequences corresponding to SA and SD comprise the splicing signal. Taking the splicing site as the boundary, in the SD sequence, for the portion related to determining the upstream splicing site, the part located in the exon may be called as A1 and the part located in the intron may be called as B1. Similarly, in the SA sequence, for the portion related to determining the downstream splicing site, the part located in the exon may be called as A2 and the part located in the intron may be called as B2. In this way, after the target gene is transcribed, the signal RNA finally formed will leave a sequence corresponding to A1A2 at the splicing junction.

The term "downstream splicing donor site (SD)" generally refers to a splicing donor (SD).

The term "intein" generally refers to a functional protein that can mediate the self-excision of a protein molecule from a precursor molecule and simultaneously link the extein proteins on both sides through peptide bonds. The intein gene may not be an independent gene, and it needs to be inserted into the extein genes for replication and transcription. It may be excised from the precursor protein, and link the exteins on both sides to form a mature protein. The nucleotide sequence corresponding to the intein may be embedded in a nucleic acid sequence corresponding to a host protein, existed in the same open reading frame with the host protein gene, and synchronously transcribed and translated with the host protein gene. After being translated into a protein precursor, the intein may be excised from the host protein so as to form a mature active protein. According to the existence form of the intein, it may be divided into an integral intein and split inteins. Two splicing regions of the integral intein are co-existed on the same polypeptide fragment, while two splicing regions of the split inteins are split into two or more fragments, and the two splicing regions are existed on different polypeptide fragments, so it may also be called as a split intein.

The term "first constant region" generally refers to a constant region that can be expressed by the first expression cassette described herein. The constant region may mean being relative to the "variable" region. The constant region can interact, such that the N-terminal truncated version of the ABCA4 protein expressed by the first expression cassette and the C-terminal truncated version of the ABCA4 protein expressed by the second expression cassette interact to form an N-C heterodimer of the ABCA4 protein. In certain cases, the constant region may comprise a protein encoded by a constant region gene of an immunoglobulin. The constant region may be selected from any of the following five isotypes: α, δ, ε, γ, or µ.

The term "second constant region" generally refers to a constant region that can be expressed by the second expression cassette described herein. As used herein, the second constant region may be the same as the first constant region, or may be different from the first constant region.

The term "knob-into-hole modification" generally refers to a modification within the interface between the CH3 domains of two immunoglobulin heavy chains. In one embodiment, the "knob-into-hole modification" comprises amino acid substitution T366W and optionally amino acid substitution S354C in one antibody heavy chain, as well as amino acid substitution T366S, L368A, Y407V, and optionally Y349C in another antibody heavy chain. The knob-into-hole technique is described in, for example, U.S. Pat. No. 8,216,805.

The term "liposome" generally refers to a lipid structure formed by amphiphilic vesicle-forming lipids. The liposome may be a closed vesicle composed of a monolayer or more lipid bilayer having an internal aqueous phase. For example, in a broader sense, a liposome may refer to a lipid composite particle. The liposome may even comprise a complex whose aqueous phase is not confirmed definitively. This lipid complex contains at least one type of lipid and may additionally contain hydrophilic polymers, polysaccharides, amino acids, etc. The lipid complex may refer to a particle formed by these components via covalent or non-covalent bonds.

The term "exosome" generally refers to a membrane vesicle involving the external secretion of a cell or having a membrane structure composed of a lipid bilayer present in a cell. The exosome may comprise a membrane body having an average diameter from about 10 nm to about 2,000 nm. The exosome may comprise a microvesicle. The microvesicle, also called as a circulating microvesicle or microparticle, is a fragment of the plasma membrane with an approximate diameter ranging from 100 nm to 1000 nm that is shed from almost all cell types. The exosome may also comprise smaller extracellular vesicles which are generated intracellularly. They are formed by inward budding of the limiting membrane of a multivesicular body (MVB), which, upon fusion with the plasma membrane, leads to their secretion and deposition in body fluids (*e.g.*, blood, urine). The exosome may contain a complex mixture of microRNAs (miR), mRNAs, and proteins, which reflects the state of transcription and translation of the producing cell.

The term "AAV" generally refers to the abbreviation of adeno-associated virus, and may be used to refer to the virus itself or its derivatives. The AAV may comprise AAV type 1 (AAV-1 or AAV1), AAV type 2 (AAV-2 or AAV2), AAV type 3 (AAV-3 or AAV3), AAV type 4 (AAV-4 or AAV4), AAV type 5 (AAV-5 or AAV5), AAV type 6 (AAV-6 or AAV6), AAV type 7 (AAV-7 or AAV7), AAV type 8 (AAV-8 or AAV8), AAV type 9 (AAV-9 or AAV9), bird AAV, bovine AAV, canine AAV, horse AAV, primate AAV, non-primate AAV, and sheep AAV.

The term "kit" generally refers to a package containing one or more active ingredients in one or more suitable containers.

The term "disease caused by an ABCA4 mutation" generally refers to a disease associated with an ABCA4 gene mutation, such as an ophthalmic rare disease.

The term "inherited retinal disease" generally refers to inherited retinal dystrophies or inherited retinal diseases (IRDs), which belong to ophthalmic rare diseases causing the loss and/or progressive degeneration of retinal function in patients due to genetic defects. The inherited retinal disease often causes reversible or irreversible visual impairments in infancy or adolescence. IRDs are refractory ophthalmic diseases, which are mostly manifested as abnormal protein metabolism. The purpose of treatment may be achieved by regulating genes, transcription, and translation in the process of genetic information transmission. The inherited retinal disease may comprise autosomal recessive retinitis pigmentosa (RP, MERTK mutation), choroideremia (CHM mutation), juvenile macular degeneration (ABCA4 mutation), Usher syndrome subtype 1B (Myo7a mutation), X-linked congenital retinoschisis (RS1 mutation), mitochondrial-related Leber hereditary optic neuropathy (ND4 mutation), achromatopsia (CNGA3 mutation and CNGB3 mutation), X-linked RP (RPGR mutation), etc.

The term "hereditary macular degeneration disease" generally refers to a rare familial hereditary disease. The hereditary macular degeneration disease may comprise Stargardt disease. Stargardt disease is an autosomal recessive disease that primarily develop in the retinal pigment epithelium, which is occurred more frequently in children of consanguineous marriages, with relatively rare sporadic cases. The patients have macular atrophic impairments combined with yellow spot depositions in retina. There is currently no approved treatment, and Stargardt disease may cause vision loss in thousands of people worldwide each year. The hereditary macular degeneration disease may also comprise Best disease, in which the lesions mainly involve the macular areas of both eyes but generally do not spread to the surrounding retina. The hereditary macular degeneration disease may occur at any age.

The term "age-related macular degeneration" generally refers to age-related macular degeneration (AMD), which is one of the main causes of blindness in people ≥ 50 years of age. AMD may be manifested as the progressive and irreversible loss of central vision, mainly involving the macula, retinal pigment epithelium, choroid and others. Clinically, early AMD is mainly characterized by subretinal drusen deposition, while late AMD is divided into two types: dry and wet. The main pathological changes in dry AMD (also called as atrophic AMD) are geographic atrophy and detachment of the RPE layer, as well as slow and progressive loss of vision in the diseased eye. The main pathological changes in wet AMD (also called as neovascular or exudative AMD) are choroidal neovascularization (CNV) as well as secondary liquid accumulation and hemorrhage. The pathogenesis of AMD is still unclear, and may involve RPE impairment, mitochondrial dysfunction, oxidative stress, inflammation, complement pathway activation, etc. At present, it is generally believed that the occurrence of AMD is a result of the combined action of genetic and environmental factors.

The term "retinitis pigmentosa" generally refers to Retinitis Pigmentosa (RP), which is a group of hereditary eye diseases characterized by the progressive degenerations of retinal photoreceptors. The retinitis pigmentosa may comprise a progressive retinal (a layer of transparent photosensitive membrane on the inner surface in the posterior eye) degenerative disease, which may eventually lead to moderate to severe vision loss. The retinal pigment lesions are mostly hereditary. The clinical manifestations of the retinitis pigmentosa may comprise early night blindness, concentric narrowing of the visual field, and eventually tunnel vision, binocular blindness, or near-blindness. It accounts for a considerable proportion of blindness-causing eye diseases. The retinitis pigmentosa may belong to a hereditary rod and cone dystrophy disease.

The term "cone-rod dystrophy" generally refers to cone-rod dystrophy (CRD), which belongs to a hereditary macular degeneration disease. The cone-rod dystrophy may comprise a group of clinical and genetic syndromes such as progressive loss of vision, photophobia, and nystagmus, mainly involving cone cells and possibly complicating rod cells in severe cases. The inheritance modes are mainly autosomal dominant, recessive, and X-linked inheritance.

The term "heterologous" generally refers to a polynucleotide, gene, or polypeptide that is not normally found in the host organism when used to mention a polynucleotide, gene, or polypeptide. The term "heterologous" may also comprise a native coding region or parts thereof, which is re-introduced into the source organism in a form different from the corresponding native gene (for example, which may not be in the native position in the genome of the organism).

The term "retinylphosphatidylethanolamine (NRPE)" generally refers to a physiological lipid substrate of ABCA4, which is sandwiched between two TMDs in the luminal leaflet and is further stabilized by an extended loop from the extracellular domain 1.

### DETAILED DESCRIPTION OF THE INVENTION

In one aspect, the present application provides an expression cassette combination, comprising a first expression cassette and a second expression cassette, wherein the first expression cassette is capable of expressing an N-terminal truncated version of an ABCA4 protein, the second expression cassette is capable of expressing a C-terminal truncated version of an ABCA4 protein, and the N-terminal truncated version of the ABCA4 protein expressed by the first expression cassette and the C-terminal truncated version of the ABCA4 protein expressed by the second expression cassette are capable of possessing the biological functions of a complete ABCA4 protein without forming the complete ABCA4 protein therefrom.

In the present application, the biological function of the complete ABCA4 protein may comprise cellular localization function; ATP hydrolase activity; expression in photoreceptor cells, and/or reducing retinal A2E deposition.

In the present application, the N-terminal truncated version of the ABCA4 protein may comprise an amino acid sequence set forth in amino acids 1-1325, amino acids 1-1160, amino acids 1-1220, amino acids 1-1280, or amino acids 1-1347 from the N-terminus of the complete human ABCA4 protein. For example, the amino acid sequence of the N-terminal truncated version of the ABCA4 protein may be as set forth in amino acids 1-1325 from the N-terminus of the complete human ABCA4 protein. In the present application, the C-terminal truncated version of the ABCA4 protein may comprise an amino acid sequence set forth in amino acids 1326-2273, amino acids 1348-2273, amino acids 1369-2273, or amino acids 1348-2170 from the N-terminus of the complete human ABCA4 protein. For example, the amino acid sequence of the C-terminal truncated version of the ABCA4 protein may be as set forth in amino acids 1326-2273 from the N-terminus of the complete human ABCA4 protein. In the present application, the N-terminal truncated version of the ABCA4 protein and/or the C-terminal truncated version of the ABCA4 protein may correspond to the blue and green structures in Figure 1 of Nature Communications volume 12, Article number: 3853 (2021), respectively.

In the present application, the complete ABCA4 protein may be composed of the N-terminal truncated version of the ABCA4 protein and the C-terminal truncated version of the ABCA4 protein. The N-terminal truncated version of the ABCA4 protein and the C-terminal truncated version of the ABCA4 protein may interact in spatial conformation (*e.g.*, to form an N-C heterodimer) due to their similar structures, and there is a possibility to form a complete ABCA4 protein through interaction. At the same time, the N-terminal truncated version of the ABCA4 protein and the C-terminal truncated version of the ABCA4 protein may also be present individually and separately, without forming the complete ABCA4 protein.

In the present application, the N-terminal truncated version of the ABCA4 protein expressed by the first expression cassette and the C-terminal truncated version of the ABCA4 protein expressed by the second expression cassette may not substantially (*e.g.*, at at least about 50% molar proportion, at least about 60% molar proportion, at least about 70% molar proportion, at least about 80% molar proportion, at least about 90% molar proportion, at least about 95% molar proportion or higher) interact to form the complete ABCA4 protein.

In the present application, the protein molar ratio of the N-terminal truncated version of the ABCA4 protein expressed by the first expression cassette to the C-terminal truncated version of the ABCA4 protein expressed by the second expression cassette is about 3:1 to 1:3. For example, the protein molar ratio of the N-terminal truncated version of the ABCA4 protein to the C-terminal truncated version of the ABCA4 protein expressed by the second expression cassette may be about 3:1 to 1:3, about 3:1 to 1:2, about 3:1 to 1:1, about 3:1 to 2:1, about 2:1 to 1:3, about 2:1 to 1:2, about 2:1 to 1:1, about 1:1 to 1:3, about 1:2 to 1:1, or about 1:1.

In the present application, the first expression cassette and/or the second expression cassette do not express a junction sequence enabling the N-terminal truncated version of the ABCA4 protein expressed by the first expression cassette and the C-terminal truncated version of the ABCA4 protein expressed by the second expression cassette to form the complete ABCA4 protein.

In the present application, the junction sequence enables the N-terminal truncated version of the ABCA4 protein expressed by the first expression cassette and the C-terminal truncated version of the ABCA4 protein expressed by the second expression cassette to form the complete ABCA4 protein through a method selected from the group consisting of homologous recombination, mRNA splicing, and protein splicing.

The N-terminal truncated version of the ABCA4 protein and the C-terminal truncated version of the ABCA4 protein may interact (*e.g.*, homologous recombination, mRNA splicing, and/or protein splicing) through the junction sequence to form the complete ABCA4 protein. In the present application, when the first expression cassette and/or the second expression cassette do not express the junction sequence, the N-terminal truncated version of the ABCA4 protein expressed by the first expression cassette and the C-terminal truncated version of the ABCA4 protein expressed by the second expression cassette may not substantially (*e.g.*, at at least about 50% molar proportion, at least about 60% molar proportion, at least about 70% molar proportion, at least about 80% molar proportion, at least about 90% molar proportion, at least about 95% molar proportion or higher) interact to form the complete ABCA4 protein.

In the present application, the junction sequence may comprise a homologous arm sequence.

In the present application, the junction sequence may comprise a sequence capable of being complementary to a sequence encoding at least a portion of the N-terminal truncated version of the ABCA4 protein; and/or the junction sequence may comprise a sequence capable of being complementary to a sequence encoding at least a portion of the C-terminal truncated version of the ABCA4 protein. For example, the homologous arm sequence may comprise an upstream homologous arm sequence and/or a downstream homologous arm sequence. The upstream homologous arm sequence may comprise a sequence capable of being complementary to a sequence encoding at least a portion of the N-terminal truncated version of the ABCA4 protein; and/or the downstream homologous arm sequence may comprise a sequence capable of being complementary to a sequence encoding at least a portion of the C-terminal truncated version of the ABCA4 protein.

In the present application, the first expression cassette cannot comprise a sequence capable of being complementary to a sequence encoding at least a portion of the N-terminal truncated version of the ABCA4 protein; and/or the second expression cassette cannot comprise a sequence capable of being complementary to a sequence encoding at least a portion of the C-terminal truncated version of the ABCA4 protein. The first expression cassette and/or the second expression cassette cannot enable the interaction between the N-terminal truncated version of the ABCA4 protein and the C-terminal truncated version of the ABCA4 protein expressed thereby through homologous recombination to form the complete ABCA4 protein.

In the present application, the junction sequence may comprise a splicing signal of mRNA intron.

For example, the splicing signal of mRNA intron may comprise an intron upstream splicing acceptor site (SA) and/or an intron downstream splicing donor site (SD). For example, the intron upstream splicing acceptor site (SA) may be located downstream of the sequence encoding at least a portion of the N-terminal truncated version of the ABCA4 protein; and/or the intron downstream splicing donor site (SD) may be located upstream of the sequence encoding at least a portion of the C-terminal truncated version of the ABCA4 protein.

In the present application, the first expression cassette cannot comprise an intron upstream splicing acceptor site (SA); and/or the second expression cassette cannot comprise an intron downstream splicing donor site (SD). The first expression cassette and/or the second expression cassette cannot enable the interaction between the N-terminal truncated version of the ABCA4 protein and the C-terminal truncated version of the ABCA4 protein expressed thereby through mRNA splicing to form the complete ABCA4 protein.

In the present application, the junction sequence may comprise an intein.

In the present application, the junction sequence may comprise a sequence encoding an intein protein. For example, the sequence encoding an intein protein may comprise a sequence encoding the N-terminal portion of the intein protein; and/or a sequence encoding the C-terminal portion of the intein protein. For example, the sequence encoding the N-terminal portion of the intein protein may be located downstream of the sequence encoding at least a portion of the N-terminal truncated version of the ABCA4 protein; and/or the sequence encoding the C-terminal portion of the intein protein may be located upstream of the sequence encoding at least a portion of the C-terminal truncated version of the ABCA4 protein.

In the present application, the first expression cassette does not express a sequence encoding the N-terminal portion of the intein protein; and/or the second expression cassette does not express a sequence encoding the C-terminal portion of the intein protein. The first expression cassette and/or the second expression cassette cannot enable the interaction between the N-terminal truncated version of the ABCA4 protein and the C-terminal truncated version of the ABCA4 protein expressed thereby through protein splicing to form the complete ABCA4 protein.

In the present application, the N-terminal truncated version of the ABCA4 protein may comprise domains at the N-terminal of the ABCA4 protein: transmembrane domains TMD1 to TMD6, extracellular domains ECD1, IH1, IH2, EH1, EH2, NBD1, and/or R1.

In the present application, the N-terminal truncated version of the ABCA4 protein may comprise, sequentially from the N-terminus, IH1, TMD1, ECD1, TMD2, IH2, TMD3, TMD4, TMD5, EH1, EH2, TMD6, NBD1, and/or R1. For example, the N-terminal truncated version of the ABCA4 protein may comprise, sequentially from the N-terminus, IH1, TMD1, ECD1, TMD2, IH2, TMD3, TMD4, TMD5, EH1, EH2, TMD6, and/or NBD1.

In the present application, the N-terminal truncated version of the ABCA4 protein may comprise an amino acid sequence set forth in SEQ ID NO: 2 or 4.

In the present application, the first expression cassette may comprise a nucleotide sequence encoding the N-terminal truncated version of the ABCA4 protein.

In the present application, the nucleotide sequence encoding the N-terminal truncated version of the ABCA4 protein may be as set forth in SEQ ID NO: 25 or 27.

In the present application, the first expression cassette may comprise a promoter. In the present application, the promoter may comprise a constitutive promoter and/or an inducible promoter. For example, the promoter may comprise a CMV promoter.

In the present application, the C-terminal truncated version of the ABCA4 protein may comprise domains at the C-terminal of the ABCA4 protein: transmembrane domains TMD7 to TMD12, extracellular domains ECD1, IH3, IH4, EH3, EH4, NBD2, and/or R2.

In the present application, the C-terminal truncated version of the ABCA4 protein may comprise, sequentially from the N-terminus, IH3, TMD7, ECD2, TMD8, IH4, TMD9, TMD10, TMD11, EH3, EH4, TMD12, NBD2, and/or R2. For example, the C-terminal truncated version of the ABCA4 protein may comprise, sequentially from the N-terminus, IH3, TMD7, ECD2, TMD8, IH4, TMD9, TMD10, TMD11, EH3, EH4, TMD12, and/or NBD2.

In the present application, the C-terminal truncated version of the ABCA4 protein may comprise an amino acid sequence set forth in SEQ ID NO: 3 or 5.

In the present application, the second expression cassette may comprise a nucleotide sequence encoding the C-terminal truncated version of the ABCA4 protein.

In the present application, the nucleotide sequence encoding the C-terminal truncated version of the ABCA4 protein may be as set forth in SEQ ID NO: 26 or 28.

In the present application, the second expression cassette may comprise a promoter.

In the present application, the first expression cassette may express a first constant region, and/or the second expression cassette may express a second constant region, wherein the first constant region and the second constant region are capable of interacting such that the N-terminal truncated version of the ABCA4 protein expressed by the first expression cassette and the C-terminal truncated version of the ABCA4 protein expressed by the second expression cassette interact to form an N-C heterodimer of the ABCA4 protein.

In the present application, the first constant region may have a first modification, and/or the second constant region may have a second modification, wherein the first modification and the second modification can promote the formation of the heterodimer.

In the present application, the first constant region and the second constant region may be derived from a constant region of an antibody. For example, they may be derived from the IgG constant region of an antibody. For example, they may be derived from the IgG1 constant region of an antibody. For example, they may be derived from human IgG constant region of an antibody.

In the present application, the first modification may be the same as the second modification. In the present application, the first modification may be different from the second modification. For example, the formation of the heterodimer may be promoted through the charge attraction of the first modification with the second modification. For example, the first modification and the second modification may comprise a knob-into-hole modification. For example, the first modification may have a knob modification and the second modification may have a hole modification; and/or the first modification may have a hole modification and the second modification may have a knob modification.

In the present application, the first constant region may comprise an amino acid sequence set forth in SEQ ID NO: 6. In the present application, the second constant region may comprise an amino acid sequence set forth in SEQ ID NO: 7.

In the present application, the first constant region may comprise an amino acid sequence set forth in SEQ ID NO: 7. In the present application, the second constant region may comprise an amino acid sequence set forth in SEQ ID NO: 6.

In the present application, R1 in the N-terminal truncated version of the ABCA4 protein may interact with R2 in the C-terminal truncated version of the ABCA4 protein to help the N-terminal truncated version of the ABCA4 protein and the C-terminal truncated version of the ABCA4 protein form the N-C heterodimer of the ABCA4 protein. In certain cases, R1 in the N-terminal truncated version of the ABCA4 protein may be replaced by the first constant region, and/or R2 in the C-terminal truncated version of the ABCA4 protein may be replaced by the second constant region, such that through the interaction of the first constant region and the second constant region, the N-terminal truncated version of the ABCA4 protein expressed by the first expression cassette and the C-terminal truncated version of the ABCA4 protein expressed by the second expression cassette interact to form the N-C heterodimer of the ABCA4 protein. In certain cases, the downstream of R1 in the N-terminal truncated version of the ABCA4 protein may be further linked to the first constant region, and/or the downstream of R2 in the C-terminal truncated version of the ABCA4 protein may be further linked to the second constant region, further promoting the interaction between the N-terminal truncated version of the ABCA4 protein expressed by the first expression cassette and the C-terminal truncated version of the ABCA4 protein expressed by the second expression cassette to form the N-C heterodimer of the ABCA4 protein.

In the present application, the first expression cassette may comprise, from the 5' end, a promoter sequence, a nucleotide sequence encoding the N-terminal truncated version of the ABCA4 protein, and/or a nucleotide sequence encoding the first constant region. In the present application, the first expression cassette may comprise, from the 5' end, a promoter sequence, a nucleotide sequence encoding the first constant region, and/or a nucleotide sequence encoding the N-terminal truncated version of the ABCA4 protein.

In the present application, the first expression cassette may be consisted of, from the 5' end, a promoter sequence and a nucleotide sequence encoding the N-terminal truncated version of the ABCA4 protein. In the present application, the first expression cassette may be consisted of, from the 5' end, a nucleotide sequence of a promoter, a nucleotide sequence encoding the first constant region, and a nucleotide sequence encoding the N-terminal truncated version of the ABCA4 protein. In the present application, the first expression cassette may be consisted of, from the 5' end, a nucleotide sequence of a promoter, a nucleotide sequence encoding the N-terminal truncated version of the ABCA4 protein, and a nucleotide sequence encoding the first constant region.

In the present application, the second expression cassette may comprise, from the 5' end, a promoter sequence, a nucleotide sequence encoding the C-terminal truncated version of the ABCA4 protein, and/or a nucleotide sequence encoding the second constant region. In the present application, the second expression cassette may comprise, from the 5' end, a promoter sequence, a nucleotide sequence encoding the second constant region, and/or a nucleotide sequence encoding the C-terminal truncated version of the ABCA4 protein.

In the present application, the second expression cassette may be consisted of, from the 5' end, a promoter sequence and a nucleotide sequence encoding the C-terminal truncated version of the ABCA4 protein. In the present application, the second expression cassette may be consisted of, from the 5' end, a nucleotide sequence of a promoter, a nucleotide sequence encoding the C-terminal truncated version of the ABCA4 protein, and a nucleotide sequence encoding the second constant region. In the present application, the second expression cassette may be consisted of, from the 5' end, a promoter sequence, a nucleotide sequence encoding the second constant region, and a nucleotide sequence encoding the C-terminal truncated version of the ABCA4 protein.

In the present application, the protein expressed by the first expression cassette may comprise, sequentially from the N-terminus, IH1, TMD1, ECD1, TMD2, IH2, TMD3, TMD4, TMD5, EH1, EH2, TMD6, NBD1, and/or R1, and the first constant region.

In the present application, the protein expressed by the first expression cassette may comprise, sequentially from the N-terminus, the first constant region, IH1, TMD1, ECD1, TMD2, IH2, TMD3, TMD4, TMD5, EH1, EH2, TMD6, NBD1, and R1.

For example, the protein expressed by the first expression cassette may be, sequentially from the N-terminus, IH1, TMD1, ECD1, TMD2, IH2, TMD3, TMD4, TMD5, EH1, EH2, TMD6, NBD1, R1, and the first constant region. For example, the protein expressed by the first expression cassette may be, sequentially from the N-terminus, IH1, TMD1, ECD1, TMD2, IH2, TMD3, TMD4, TMD5, EH1, EH2, TMD6, NBD1, and the first constant region. For example, the protein expressed by the first expression cassette may be, sequentially from the N-terminus, the first constant region, IH1, TMD1, ECD1, TMD2, IH2, TMD3, TMD4, TMD5, EH1, EH2, TMD6, NBD1, and R1. For example, the protein expressed by the first expression cassette may be, sequentially from the N-terminus, the first constant region, IH1, TMD1, ECD1, TMD2, IH2, TMD3, TMD4, TMD5, EH1, EH2, TMD6, and NBD1.

In the present application, the protein expressed by the second expression cassette may comprise, sequentially from the N-terminus, IH3, TMD7, ECD2, TMD8, IH4, TMD9, TMD10, TMD11, EH3, EH4, TMD12, NBD2, and/or R2, and the second constant region.

In the present application, the protein expressed by the second expression cassette may comprise, sequentially from the N-terminus, the second constant region, IH3, TMD7, ECD2, TMD8, IH4, TMD9, TMD10, TMD11, EH3, EH4, TMD12, and NBD2 and/or R2.

For example, the protein expressed by the second expression cassette may be, sequentially from the N-terminus, IH3, TMD7, ECD2, TMD8, IH4, TMD9, TMD10, TMD11, EH3, EH4, TMD12, NBD2, R2, and the second constant region. For example, the protein expressed by the second expression cassette may be, sequentially from the N-terminus, IH3, TMD7, ECD2, TMD8, IH4, TMD9, TMD10, TMD11, EH3, EH4, TMD12, NBD2, and the second constant region. For example, the protein expressed by the second expression cassette may be, sequentially from the N-terminus, the second constant region, IH3, TMD7, ECD2, TMD8, IH4, TMD9, TMD10, TMD11, EH3, EH4, TMD12, NBD2, and R2. For example, the protein expressed by the second expression cassette may be, sequentially from the N-terminus, the second constant region, IH3, TMD7, ECD2, TMD8, IH4, TMD9, TMD10, TMD11, EH3, EH4, TMD12, and NBD2.

In the present application, the first expression cassette may comprise a nucleotide sequence set forth in any of SEQ ID NOs: 2, 4, 8-10, 15-18, and 34.

In the present application, the second expression cassette may comprise a nucleotide sequence set forth in any of SEQ ID NOs: 3, 5, 11-14, and 19-22.

In the present application, the first expression cassette and the second expression cassette may be present individually.

In the present application, the first expression cassette and/or the second expression cassette may be present in the form selected from the group consisting of: an isolated nucleic acid molecule, a liposome, and/or an exosome.

In the present application, the first expression cassette and/or the second expression cassette may be present in the form of a plasmid.

In the present application, the first expression cassette and/or the second expression cassette may be a viral vector.

In the present application, the first expression cassette and/or the second expression cassette may be AAV.

In the present application, in the expression cassette combination, the molar ratio of the protein expressed by the first expression cassette to the protein expressed by the second expression cassette may be about 1:3 to 3:1. For example, it may be about 3:1 to 1:3, about 3:1 to 1:2, about 3:1 to 1:1, about 3:1 to 2:1, about 2:1 to 1:3, about 2:1 to 1:2, about 2:1 to 1:1, about 1:1 to 1:3, about 1:2 to 1:1, or about 1:1.

In the present application, in the expression cassette combination, the molar ratio of the protein expressed by the first expression cassette to the protein expressed by the second expression cassette may be about 1:1.

In another aspect, the present application provides a kit comprising the expression cassette combination described in the present application.

In the present application, the kit may comprise reagents and/or instruments for administrating the expression cassette combination. For example, the kit may comprise reagents and/or instruments for the transfection of AAV that comprises the first expression cassette and/or the second expression cassette. For example, the kit may comprise reagents and/or instruments for injecting AAV that comprises the first expression cassette and/or the second expression cassette. In the present application, the injection may comprise topical injection.

In another aspect, the present application provides the use of the expression cassette combination described in the present application and/or the kit described in the present application in the manufacture of a medicament for treating a disease, wherein the disease comprises a disease caused by an ABCA4 mutation.

In the present application, the disease may comprise an inherited retinal disease.

In the present application, the disease may comprise a hereditary macular degeneration disease, age-related macular degeneration, retinitis pigmentosa, and/or cone-rod dystrophy.

With the method described in the present application, a heterologous N-terminal truncated version of the ABCA4 protein and a heterologous C-terminal truncated version of the ABCA4 protein can be expressed in a subject in need thereof. Since the N-terminal truncated version of the ABCA4 protein and the C-terminal truncated version of the ABCA4 protein (heterologous) can exert the biological functions of the complete ABCA4 protein, they can be used to alleviate, treat, and/or ameliorate the disease caused by an ABCA4 mutation.

In another aspect, the present application provides a method for expressing a heterologous ABCA4 gene, which may comprise administrating the expression cassette combination described in the present application and/or the kit described in the present application to a subject in need thereof.

In another aspect, the present application provides a method for alleviating the cell death caused by retinylphosphatidylethanolamine (NRPE), which may comprise administrating the expression cassette combination described in the present application and/or the kit described in the present application to a subject in need thereof.

In another aspect, the present application provides a method for treating a disease caused by an ABCA4 mutation, which may comprises administrating the expression cassette combination described in the present application and/or the kit described in the present application to a subject in need thereof.

In the present application, the administrating may comprise injection.

In the present application, the disease may comprise an inherited retinal disease.

In the present application, the disease may comprise a hereditary macular degeneration disease, age-related macular degeneration, retinitis pigmentosa, and/or cone-rod dystrophy.

Without intention to be limited by any theory, the following Examples are only intended to illustrate the expression cassettes, preparation methods, uses, etc. in the present application, and are not intended to limit the scope of the claimed invention.

### Examples

### Example 1 Improved ABCA4 protein expression by codon-optimization

The wild-type ABCA4 plasmid (ABCA4-WT) fused with a FLAG tag or the codon-optimized ABCA4 plasmid (ABCA4-CO) fused with a FLAG tag was respectively transfected into HEK293 cells (provided by Peking University Health Science Center). After 48 hours, the cells were lysed, and the expression of ABCA4 proteins in the cells was detected by using an endogenous antibody (NBP1-30032, NovusBio) or a flag tag antibody (F1804, Sigma) for ABCA4 respectively. Among them, the nucleotide sequence of wild-type ABCA4 was as set forth in SEQ ID NO. 23; and the nucleotide sequence of codon-optimized ABCA4 was as set forth in SEQ ID NO. 24.

The Western results showed that (*see* FIG. 2), the expression of the codon-optimized ABCA4 (ABCA4-CO) was significantly higher than that of wild-type ABCA4 (ABCA4-WT).

### Example 2 Homodimerization of the N-terminal truncated version and the C-terminal truncated version of ABCA4

Two truncated versions of ABCA4, namely the N-terminal truncated version (aa 1-1347) and the C-terminal truncated version (aa 1348-2273), were respectively constructed by using the full-length CDS sequence of codon-optimized ABCA4 (aa 1-2273) and dividing according to the structural functional domains of the ABCA4 protein (Scortecci, J. F., et al. (2021). Nat Commun 12(1): 5902.; Xie, T., et al. (2021). Nat Commun 12(1): 3853). Among them, the full-length amino acid sequence of ABCA4 was as set forth in SEQ ID NO. 1; the amino acid sequence of the N-terminal truncated version of ABCA4 was as set forth in SEQ ID NO. 2; and the amino acid sequence of the C-terminal truncated version of ABCA4 was as set forth in SEQ ID NO. 3.

To facilitate the protein detection, a hemagglutinin (HA) tag was linked to the C-terminus of the N-terminal truncated version, and a Flag tag was linked to the C-terminus of the C-terminal truncated version.

The expression plasmids for the full-length ABCA4, the N-terminal truncated version, or the C-terminal truncated version were transfected into HEK293 cells, and then the respective tags were labeled with respective antibodies.

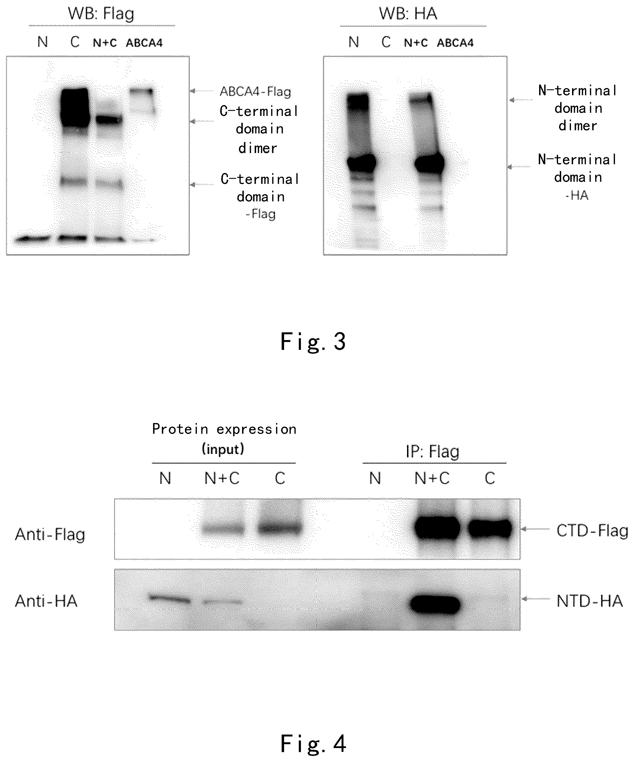

The Western results showed that (*see* FIG. 3), there was homodimerization of the N-terminal truncated version or the C-terminal truncated version of ABCA4, and the co-transfection of the N-terminal truncated version and the C-terminal truncated version could reduce the proportion of homodimerization.

### Example 3 Interaction between the N-terminal truncated version and the C-terminal truncated version of ABCA4

The N-terminal truncated version (aa 1-1347) and the C-terminal truncated version (aa 1348-2273) of ABCA4 were respectively transfected into HEK293 cells, or N+C was co-transfected (*i.e.*, the N-terminal truncated version (aa 1-1347) and the C-terminal truncated version (aa 1348-2273) were co-transfected) into HEK293 cells. The skeleton vector for the transfected plasmid was purchased from Addgene.

After 48 hours, the cells were lysed. The C-terminal truncated version containing a flag tag in the cells was enriched by using flag antibody (F1804, Sigma)-coupled magnetic beads, and then the presence of the N-terminal truncated version interacting with the C-terminal truncated version was detected by western blot using an HA antibody (AE036, ABclonal).

The results were shown in FIG. 4. There were respective protein expressions (inputs) in HEK293 cells transfected with the N-terminal truncated version, transfected with the C-terminal truncated version, or co-transfected with N+C respectively. The C-terminal truncated version containing the flag tag in the cell lysate could be enriched by flag antibody-coupled magnetic beads, while the N-terminal truncated version interacting with the C-terminal truncated version was also enriched. Thus, there was an interaction between the N-terminal truncated version (aa 1-1347) and the C-terminal truncated version (aa 1348-2273) of ABCA4.

### Example 4 Interaction between the N-terminal truncated versions of ABCA4 and the C-terminal truncated versions of ABCA4 with different lengths or containing different domains

The N-terminal truncated versions and the C-terminal truncated versions with different lengths or containing different domains were designed by using the full-length CDS sequence of codon-optimized ABCA4, wherein the N-terminal truncated versions comprised: N1347 (aa 1-1347) (SEQ ID NO: 2), N1160 (aa 1-1160) (SEQ ID NO: 35), N1220 (aa 1-1220) (SEQ ID NO: 36), N1280 (aa 1-1280) (SEQ ID NO: 37), N1280* (aa 1-1280*, 2253-2273) (SEQ ID NO: 38); and the C-terminal truncated versions comprised: C (aa 1348-2273) (SEQ ID NO: 3), C* (aa 1-20, 1369-2273) (SEQ ID NO: 39). Among them, an HA tag was linked to the C-terminus of the N-terminal truncated version, and a Flag tag was linked to the C-terminus of the C-terminal truncated version.

The plasmids for the N-terminal truncated versions and the C-terminal truncated versions as above were combined in pairs, and then co-transfected into HEK293 cells. After 48 hours, the cells were lysed. The C-terminal truncated version containing the flag tag in the cells was enriched by using flag antibody-coupled magnetic beads, and then the presence of the N-terminal truncated version interacting with the C-terminal truncated version was detected by western blot using an HA antibody.

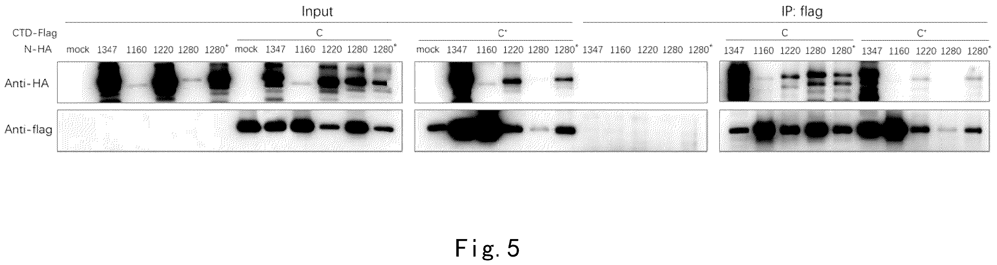

The results were shown in FIG. 5. There was an interaction between the N-terminal truncated versions and the C-terminal truncated versions of ABCA4 with different lengths or containing different domains. The interaction between each of N1347 (aa 1-1347), N1160 (aa 1-1160), N1220 (aa 1-1220), N1280 (aa 1-1280), or N1280* (aa 1-1280*, 2253-2273) and C (aa 1348-2273) could be detected; and the interaction between N1347 (aa 1-1347), N1220 (aa 1-1220), or N1280* (aa 1-1280*, 2253-2273) and C* (aa 1-20, 1369-2273) could be detected. Among them, the interaction between N1347 (aa 1-1347) and C or C* was the strongest.

### Example 5 Cellular co-localization between the N-terminal truncated version and the C-terminal truncated version of ABCA4, with the localization consistent with that of the full-length wild-type ABCA4

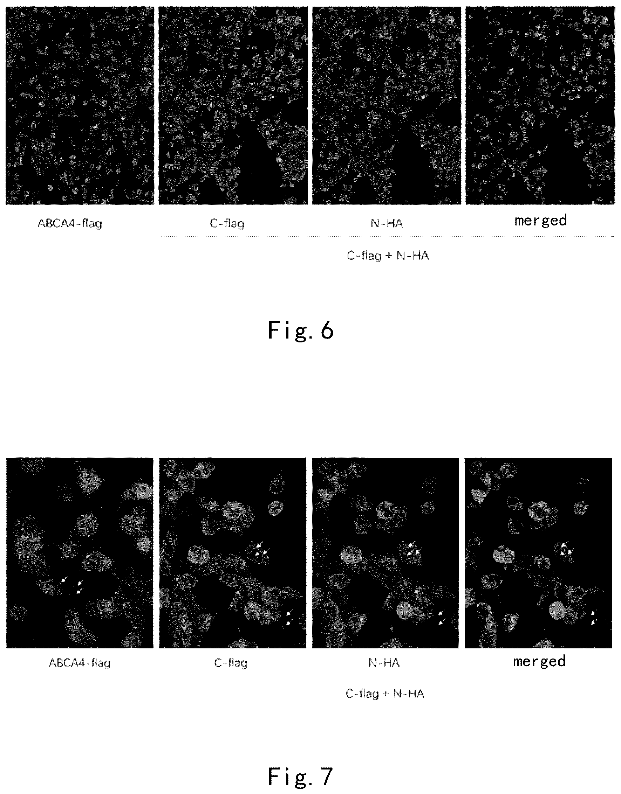

The full-length ABCA4 (aa 1-2273), the N-terminal truncated version of ABCA4 (aa 1-1347), and the C-terminal truncated version of ABCA4 (aa 1348-2273) were respectively transfected into HEK293 cells (wherein an HA tag was linked to the C-terminus of the N-terminal truncated version, and a Flag tag was linked to the C-terminus of the C-terminal truncated version), and then the respective tags were labeled with respective antibodies (a flag antibody (F1804, Sigma), an HA antibody (K200003M, Solarbio)). It could be observed that there was cellular co-localization between the N-terminal truncated version (aa 1-1347) and the C-terminal truncated version (aa 1348-2273) of ABCA4 (*see* FIG. 6), and the localization position was consistent with that of the full-length wild-type ABCA4, both of which were present in intracellular vesicles (*see* FIG. 7).

### Example 6 ATP hydrolase activity of the complex of the N-terminal truncated version and the C-terminal truncated version of ABCA4

The full-length ABCA4 (aa 1-2273), the N-terminal truncated version of ABCA4 (aa 1-1347), and the C-terminal truncated version of ABCA4 (aa 1348-2273) were transfected into HEK293 cells respectively, or N+C was co-transfected into HEK293 cells. After 48 hours, the cells were lysed. The full-length ABCA4 or the C-terminal truncated version or N+C complex (*i.e.*, the complex of the N-terminal truncated version and the C-terminal truncated version) containing a flag tag in the cells was enriched by using flag antibody-coupled magnetic beads, and then the ATP hydrolase activity of the proteins enriched on the magnetic beads was detected by using the ATPase detection kit (Promega).

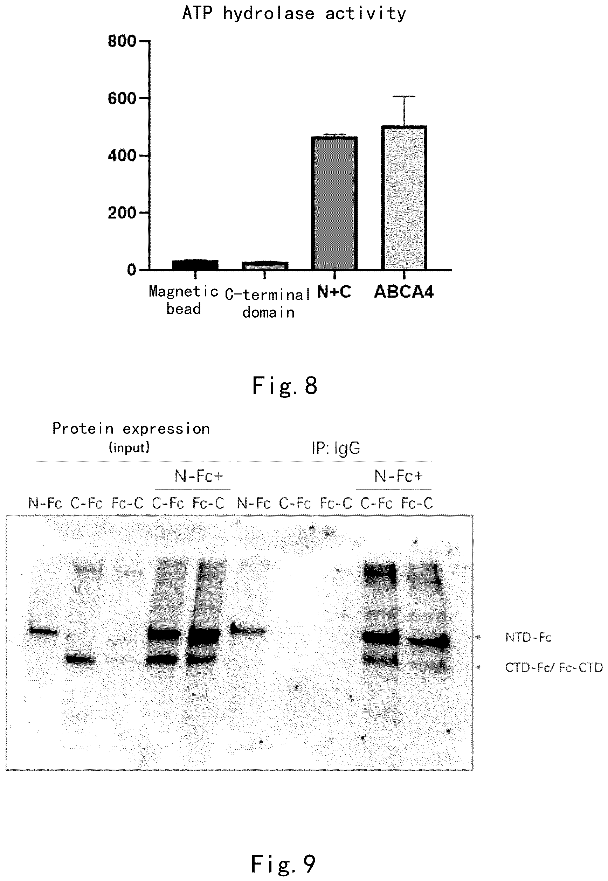

The results were shown in FIG. 8. Both the blank magnetic beads and the single C-terminal truncated version (aa 1348-2273) did not have the ATP hydrolase activity, while the N+C complex had the ATP hydrolase activity, similar to the full-length ABCA4.

### Example 7 Interaction between N (aa 1-1347)-Fca and Fcb-C (aa 1348-2273) or C (aa 1348-2273)-Fcb of ABCA4

Due to the high similarity between the N-terminal truncated version and the C-terminal truncated version of ABCA4, a structural basis is provided for the formation of a heterodimer betweenfrom, but there is also the possibility to form a homodimer.

In order to increase the proportion of the heterodimer and reduce the proportion of the homodimer, in accordance with the "knob-into-hole" principle, Fca and Fcb were used to increase the proportion of the heterodimer and reduce the proportion of the homodimer.

The amino acid sequence of Fca was as set forth in SEQ ID NO. 6; and the amino acid sequence of Fcb was as set forth in SEQ ID NO. 7.

The following two strategies were employed to link Fc to the N-terminal truncated version (aa 1-1347) and the C-terminal truncated version (aa 1348-2273) of ABCA4:
As the first strategy, Fca and Fcb were adjacent in the secondary structure of amino acid, namely N (aa 1-1347)-Fca was constructed by linking Fca to the C-terminus of the N-terminal truncated version (aa 1-1347) of ABCA4 (adjacent to R1) (where the amino acid sequence of N (aa 1-1347)-Fca was as set forth in SEQ ID NO. 8), and Fcb-C (aa 1348-2273) was constructed by linking Fcb to the N-terminus of the C-terminal truncated version (aa 1348-2273) of ABCA4 (adjacent to IH3) (where the amino acid sequence of Fcb-C (aa 1348-2273) was as set forth in SEQ ID NO. 14).

As the second strategy, Fca and Fcb were adjacent in the tertiary structure of protein, namely N (aa 1-1347)-Fca was constructed by linking Fca to the C-terminus of the N-terminal truncated version (aa 1-1347) of ABCA4 (adjacent to R1) (where the amino acid sequence of N (aa 1-1347)-Fca was as set forth in SEQ ID NO. 8), and C (aa 1348-2273)-Fcb was constructed by linking Fcb to the C-terminus of the C-terminal truncated version (aa 1348-2273) of ABCA4 (adjacent to R2) (where the amino acid sequence of C (aa 1348-2273)-Fcb was as set forth in SEQ ID NO. 13).

N (aa 1-1347)-Fca and Fcb-C (aa 1348-2273), or N (aa 1-1347)-Fca and C (aa 1348-2273)-Fcb, were co-transfected into HEK293 cells respectively. After 48 hours, the cells were lysed. N (aa 1-1347)-Fca containing the Fca tag in the cells was enriched by using IgG-coupled magnetic beads, and then the presence of Fcb-C (aa 1348-2273) or C (aa 1348-2273)-Fcb interacting with N (aa 1-1347)-Fca was detected by western blot using an anti-human antibody (AS002, ABclonal).

The results were shown in FIG. 9. There were respective protein expressions (inputs) in HEK293 cells singly transfected with or co-transfected with N (aa 1-1347)-Fca and Fcb-C (aa 1348-2273) or C (aa 1348-2273)-Fcb respectively. N (aa 1-1347)-Fca in the cell lysate could be enriched by IgG-coupled magnetic beads, while Fcb-C (aa 1348-2273) or C (aa 1348-2273)-Fcb interacting with N (aa 1-1347)-Fca was also enriched. In FIG. 9, N-Fc indicated single transfection of N (aa 1-1347)-Fca; C-Fc indicated single transfection of C (aa 1348-2273)-Fcb; Fc-C indicated single transfection of Fcb-C (aa 1348-2273); N (aa 1-1347)-Fc + C-Fc indicated co-transfection of N (aa 1-1347)-Fca and C (aa 1348-2273)-Fcb; N (aa 1-1347)-Fc + Fc-C indicated co-transfection of N (aa 1-1347)-Fca and Fcb-C (aa 1348-2273); NTD-Fc indicated enriched N (aa 1-1347)-Fca; and CTD-Fc/Fc-CTD indicated Fcb-C (aa 1348-2273) or C (aa 1348-2273)-Fcb interacting with N (aa 1-1347)-Fca.

Thus, there was an interaction between N (aa 1-1347)-Fca and Fcb-C (aa 1348-2273) or C (aa 1348-2273)-Fcb of ABCA4.

### Example 8 Interaction between the N-terminal truncated versions-Fca and the Fcb-C-terminal truncated versions or the C-terminal truncated versions-Fcb of ABCA4 with different lengths or containing different domains

The N-terminal truncated versions-Fca and the Fcb-C-terminal truncated versions or the C-terminal truncated versions-Fcb of ABCA4 with different lengths or containing different domains were constructed, wherein the N-terminal truncated versions comprised: N (aa 1-1347)-Fca, and N (aa 1-1160)-Fca; and the C-terminal truncated versions comprised: C (aa 1348-2273)-Fcb, C (aa 1348-2170)-Fcb, and Fcb-C (aa 1348-2273).

The N-terminal truncated versions and the C-terminal truncated versions of ABCA4 as above were co-transfected into HEK293 cells respectively. After 48 hours, the cells were lysed. The N-terminal truncated version containing the Fca tag in the cells was enriched by using IgG-coupled magnetic beads, and then the presence of the C-terminal truncated version interacting with the N-terminal truncated version was detected by western blot using an anti-human antibody (AS002, ABclonal).

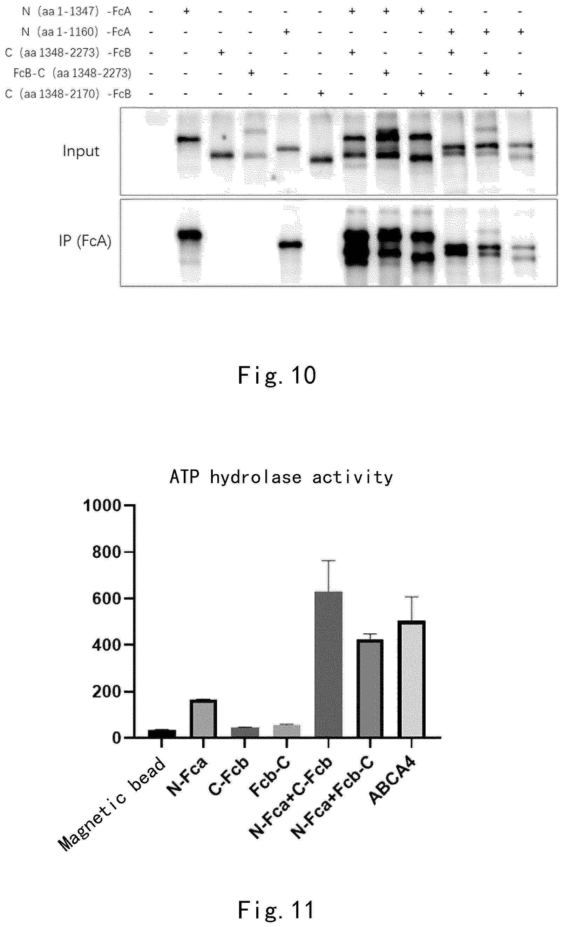

The results were shown in FIG. 10. There was a relatively strong interaction between N (aa 1-1347)-Fca or N (aa 1-1160)-Fca and C (aa 1348-2273)-Fcb, C (aa 1348-2170)-Fcb, or Fcb-C (aa 1348-2273) of ABCA4.

### Example 9 ATP hydrolase activity of the complex of N (aa 1-1347)-Fca and Fcb-C (aa 1348-2273) or N (aa 1-1347)-Fca and C (aa 1348-2273)-Fcb of ABCA4

N (aa 1-1347)-Fca and Fcb-C (aa 1348-2273), or N (aa 1-1347)-Fca and C (aa 1348-2273)-Fcb, were co-transfected into HEK293 cells respectively. After 48 hours, the cells were lysed. N (aa 1-1347)-Fca containing the Fca tag in the cells was enriched by using IgG-coupled magnetic beads (IgG-coupled magnetic beads could not bind to Fcb, *see* FIG. 11), and then the ATP hydrolase activity of the proteins enriched on the beads was detected by using the ATPase detection kit (Promega).

The results were shown in FIG. 11. The results showed that both the blank magnetic beads and the single N (aa 1-1347)-Fca did not have the ATP hydrolase activity, while the N+C complex (*i.e.*, the complex of the N-terminal truncated version and the C-terminal truncated version, which could be obtained by co-transfection of N (aa 1-1347)-Fca and Fcb-C (aa 1348-2273), or co-transfection of N (aa 1-1347)-Fca and C (aa 1348-2273)-Fcb)) had the ATP hydrolase activity, similar to the full-length ABCA4 upon enrichment via Flag-coupled magnetic beads.

### Example 10 Alleviation of cell death caused by NRPE by the complex of the N-terminal truncated version and the C-terminal truncated version of ABCA4

The negative control plasmid RHO (synthesized by Genewiz) and the full-length plasmid ABCA4 were transfected into 661W cells (provided by Peking University Health Science Center), or the N-terminal truncated version (aa 1-1347) and the C-terminal truncated version (aa 1348-2273) of ABCA4 were co-transfected into 661W cells, respectively. After 48 hours, the cells were treated with 30 µM to 50 µM of all-trans retinaldehyde (ATR) or retinylphosphatidylethanolamine (NRPE) (Aladdin). After 24 hours, the cell vitality was detected by using CellTiter-Glo (Promega).

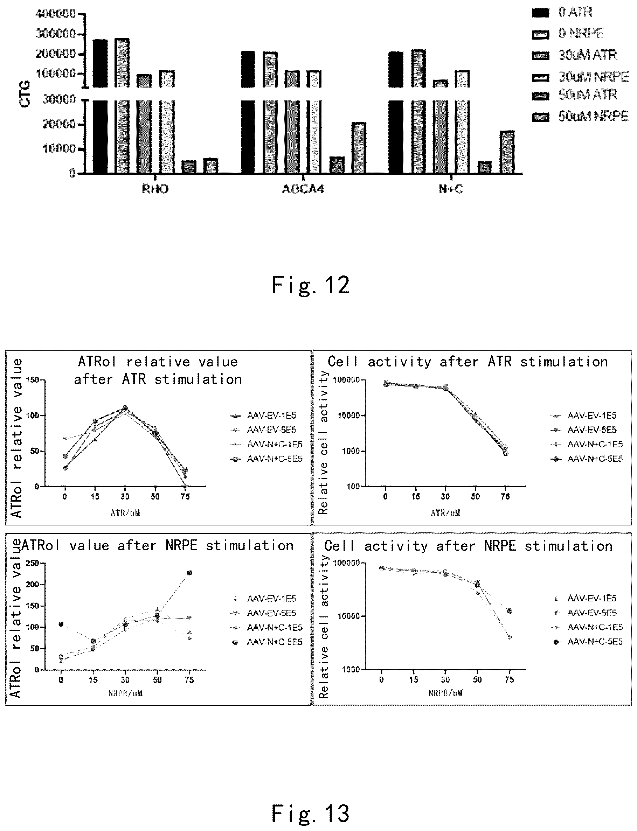

The results were shown in FIG. 12. As a high concentration of ATR or NRPE was cytotoxic, 30 µM to 50 µM of ATR or NRPE was used to stimulate for 24 hours. It could be seen that, in the RHO cell group transfected with the negative control, the cell vitality was significantly decreased (at 50 µM, the remained cell vitality was about 3%).

For the group transfected with the full-length ABCA4 or co-transfected with the N-terminal truncated version (aa 1-1347) and the C-terminal truncated version (aa 1348-2273) of ABCA4, after 30 µM to 50 µM of ATR was used to stimulate for 24 hours, the cell vitality was decreased similarly to the RHO group (at 50 µM, the remained cell vitality was about 3%); while after 30 µM to 50 µM of NRPE was used to stimulate for 24 hours, the cell vitality in each group was restored to a certain extent (at 50 µM, the remained cell vitality was about 10%).

It was shown that the expression of the full-length ABCA4 or the co-expression of the N-terminal truncated version (aa 1-1347) and the C-terminal truncated version (aa 1348-2273) of ABCA4 could promote the transportation or conversion of the substrate NRPE, thereby reducing the cytotoxicity of NRPE.

### Example 11 Alleviation of cell death caused by NRPE by the co-infection of the complex of AAV-ABCA4 N-terminal truncated version and AAV-ABCA4 C-terminal truncated version

The control viruses AAV-EV with different MOIs (MOI = 1E5 or 5E5) were infected into 661W cells, or AAV-ABCA4 N-terminal truncated version (aa 1-1347) and AAV-ABCA4 C-terminal truncated version (aa 1348-2273) were co-infected into 661W cells. After 48 hours, the cells were treated with 0 µM to 75 µM of all-trans retinaldehyde (ATR) or retinylphosphatidylethanolamine (NRPE). After 24 hours, all-trans retinol (ATRol) produced via conversion in the cells was measured by using a microplate reader, and the cell vitality was detected by CellTiter-Glo (Promega).

The results were shown in FIG. 13. After the cells were treated with a high concentration of ATR, the ATRol conversion ability in the cells was decreased, and the cell vitality was decreased. There was no difference between the group infected with the control virus AAV-EV and the group co-infected with AAV-ABCA4 N-terminal truncated version (aa 1-1347) and AAV-ABCA4 C-terminal truncated version (aa 1348-2273). After the cells were treated with a high concentration of NRPE, in the cells infected with the control virus AAV-EV, the ATRol conversion ability was decreased, and the cell vitality was decreased; while in the cells co-infected with AAV-ABCA4 N-terminal truncated version (aa 1-1347) and AAV-ABCA4 C-terminal truncated version (aa 1348-2273) with a high multiplicity of infection (MOI=5E5), the ATRol conversion ability was improved, and the cell vitality was relatively good.

It was shown that the co-infection of AAV-ABCA4 N-terminal truncated version (aa 1-1347) and AAV-ABCA4 C-terminal truncated version (aa 1348-2273) in 661W cells could promote the transportation and the conversion into ATRol of the substrate NRPE, thereby reducing the cytotoxicity of NRPE and improving the cell vitality.

All documents mentioned herein are incorporated as references in the present application, as if each is incorporated as a reference separately. In addition, it should be understood that, after reading the above teachings of the present application, those skilled in the art can make various changes or modifications to the present application, and these equivalents also fall within the scope defined by the appended claims.

## Claims

1. An expression cassette combination, comprising a first expression cassette and a second expression cassette, wherein the first expression cassette is capable of expressing an N-terminal truncated version of an ABCA4 protein, the second expression cassette is capable of expressing a C-terminal truncated version of the ABCA4 protein, and the N-terminal truncated version of the ABCA4 protein expressed by the first expression cassette and the C-terminal truncated version of the ABCA4 protein expressed by the second expression cassette are capable of possessing the biological functions of a complete ABCA4 protein without forming the complete ABCA4 protein.

2. The expression cassette combination according to claim 1, wherein the N-terminal truncated version of the ABCA4 protein expressed by the first expression cassette and the C-terminal truncated version of the ABCA4 protein expressed by the second expression cassette basically not interact to form the complete ABCA4 protein.

3. The expression cassette combination according to any of claims 1-2, wherein the protein molar ratio of the N-terminal truncated version of the ABCA4 protein expressed by the first expression cassette to the C-terminal truncated version of the ABCA4 protein expressed by the second expression cassette is about 3:1 to 1:3.

4. The expression cassette combination according to any of claims 1-3, wherein the first expression cassette and/or the second expression cassette do not express a junction sequence enabling the N-terminal truncated version of the ABCA4 protein expressed by the first expression cassette and the C-terminal truncated version of the ABCA4 protein expressed by the second expression cassette to form the complete ABCA4 protein.

5. The expression cassette combination according to claim 4, wherein the junction sequence enables the N-terminal truncated version of the ABCA4 protein expressed by the first expression cassette and the C-terminal truncated version of the ABCA4 protein expressed by the second expression cassette to form the complete ABCA4 protein through a method selected from the group consisting of homologous recombination, mRNA splicing, and protein splicing.

6. The expression cassette combination according to any of claims 4-5, wherein the junction sequence comprises a homologous arm sequence.

7. The expression cassette combination according to any of claims 4-6, wherein the junction sequence comprises a sequence capable of being complementary to a sequence encoding at least a portion of the N-terminal truncated version of the ABCA4 protein; and/or the junction sequence comprises a sequence capable of being complementary to a sequence encoding at least a portion of the C-terminal truncated version of the ABCA4 protein.

8. The expression cassette combination according to any of claims 1-7, wherein the first expression cassette does not comprise a sequence capable of being complementary to a sequence encoding at least a portion of the N-terminal truncated version of the ABCA4 protein; and/or the second expression cassette does not comprise a sequence capable of being complementary to a sequence encoding at least a portion of the C-terminal truncated version of the ABCA4 protein.

9. The expression cassette combination according to any of claims 4-8, wherein the junction sequence comprises a splicing signal of mRNA intron.

10. The expression cassette combination according to any of claims 1-9, wherein the first expression cassette does not comprise an intron upstream splicing acceptor site (SA); and/or the second expression cassette does not comprise an intron downstream splicing donor site (SD).

11. The expression cassette combination according to any of claims 4-10, wherein the junction sequence comprises an intein.

12. The expression cassette combination according to any of claims 4-11, wherein the junction sequence comprises a sequence encoding an intein protein.

13. The expression cassette combination according to any of claims 1-12, wherein the first expression cassette does not express a sequence encoding the N-terminal portion of the intein protein; and/or the second expression cassette does not express a sequence encoding the C-terminal portion of the intein protein.

14. The expression cassette combination according to any of claims 1-13, wherein the N-terminal truncated version of the ABCA4 protein comprises N-terminal domains of the ABCA4 protein: transmembrane domains TMD1 to TMD6, extracellular domains ECD1, IH1, IH2, EH1, EH2, NBD1, and/or R1, preferably transmembrane domains TMD1 to TMD6, extracellular domains ECD1, IH2, EH1, EH2, and NBD1, and optionally IH1 and/or R1.

15. The expression cassette combination according to any of claims 1-14, wherein the N-terminal truncated version of the ABCA4 protein comprises, sequentially from the N-terminus, IH1, TMD1, ECD1, TMD2, IH2, TMD3, TMD4, TMD5, EH1, EH2, TMD6, NBD1, and/or R1.

16. The expression cassette combination according to any of claims 1-15, wherein the N-terminal truncated version of the ABCA4 protein comprises an amino acid sequence set forth in SEQ ID NO: 2 or 4.

17. The expression cassette combination according to any of claims 1-16, wherein the first expression cassette comprises a nucleotide sequence encoding the N-terminal truncated version of the ABCA4 protein.

18. The expression cassette combination according to claim 17, wherein the nucleotide sequence encoding the N-terminal truncated version of the ABCA4 protein is set forth in SEQ ID NO: 25 or 27.

19. The expression cassette combination according to any of claims 1-18, wherein the first expression cassette comprises a promoter.

20. The expression cassette combination according to any of claims 1-19, wherein the C-terminal truncated version of the ABCA4 protein comprises C-terminal domains of the ABCA4 protein: transmembrane domains TMD7 to TMD12, extracellular domains ECD2, IH3, IH4, EH3, EH4, NBD2, and/or R2, preferably transmembrane domains TMD7 to TMD12, extracellular domains ECD2, IH4, EH3, EH4, and NBD2, and optionally IH3 and/or R2.

21. The expression cassette combination according to any of claims 1-20, wherein the C-terminal truncated version of the ABCA4 protein comprises, sequentially from the N-terminus, IH3, TMD7, ECD2, TMD8, IH4, TMD9, TMD10, TMD11, EH3, EH4, TMD12, NBD2, and/or R2.

22. The expression cassette combination according to any of claims 1-21, wherein the C-terminal truncated version of the ABCA4 protein comprises an amino acid sequence set forth in SEQ ID NO: 3 or 5.

23. The expression cassette combination according to any of claims 1-22, wherein the second expression cassette comprises a nucleotide sequence encoding the C-terminal truncated version of the ABCA4 protein.

24. The expression cassette combination according to claim 23, wherein the nucleotide sequence encoding the C-terminal truncated version of the ABCA4 protein is set forth in SEQ ID NO: 26 or 28.

25. The expression cassette combination according to any of claims 1-24, wherein the second expression cassette comprises a promoter.

26. The expression cassette combination according to any of claims 1-25, wherein the first expression cassette expresses a first constant region, and/or the second expression cassette expresses a second constant region, wherein the first constant region and the second constant region are capable of interacting such that the N-terminal truncated version of the ABCA4 protein expressed by the first expression cassette and the C-terminal truncated version of the ABCA4 protein expressed by the second expression cassette interact to form an N-C heterodimer of the ABCA4 protein.

27. The expression cassette combination according to claim 26, wherein the first constant region has a first modification, and/or the second constant region has a second modification, wherein the first modification and the second modification are capable of promoting the formation of the heterodimer.

28. The expression cassette combination according to any of claims 26-27, wherein the first constant region and the second constant region are derived from a constant region of an antibody.

29. The expression cassette combination according to any of claims 27-28, wherein the first modification and the second modification comprise a knob-into-hole modification.

30. The expression cassette combination according to any of claims 26-29, wherein the first constant region comprises an amino acid sequence set forth in SEQ ID NO: 6 or 7.

31. The expression cassette combination according to any of claims 26-30, wherein the second constant region comprises an amino acid sequence set forth in SEQ ID NO: 6 or 7.

32. The expression cassette combination according to any of claims 26-31, wherein the first expression cassette comprises, from the 5' end, a promoter sequence, a nucleotide sequence encoding the N-terminal truncated version of the ABCA4 protein, and/or a nucleotide sequence encoding the first constant region.

33. The expression cassette combination according to any of claims 19-32, wherein the first expression cassette is consisted of, from the 5' end, a promoter sequence and a nucleotide sequence encoding the N-terminal truncated version of the ABCA4 protein.

34. The expression cassette combination according to any of claims 26-33, wherein the first expression cassette is consisted of, from the 5' end, a nucleotide sequence of a promoter, a nucleotide sequence encoding the first constant region, and a nucleotide sequence encoding the N-terminal truncated version of the ABCA4 protein.

35. The expression cassette combination according to any of claims 26-34, wherein the first expression cassette is consisted of, from the 5' end, a nucleotide sequence of a promoter, a nucleotide sequence encoding the N-terminal truncated version of the ABCA4 protein, and a nucleotide sequence encoding the first constant region.

36. The expression cassette combination according to any of claims 26-35, wherein the second expression cassette comprises, from the 5' end, a promoter sequence, a nucleotide sequence encoding the C-terminal truncated version of the ABCA4 protein, and/or a nucleotide sequence encoding the second constant region.

37. The expression cassette combination according to any of claims 25-36, wherein the second expression cassette is consisted of, from the 5' end, a promoter sequence and a nucleotide sequence encoding the C-terminal truncated version of the ABCA4 protein.

38. The expression cassette combination according to any of claims 26-37, wherein the second expression cassette is consisted of, from the 5' end, a nucleotide sequence of a promoter, a nucleotide sequence encoding the C-terminal truncated version of the ABCA4 protein, and a nucleotide sequence encoding the second constant region.

39. The expression cassette combination according to any of claims 26-38, wherein the second expression cassette is consisted of, from the 5' end, a promoter sequence, a nucleotide sequence encoding the second constant region, and a nucleotide sequence encoding the C-terminal truncated version of the ABCA4 protein.

40. The expression cassette combination according to any of claims 1-39, wherein the first expression cassette comprises a nucleotide sequence set forth in any of SEQ ID NOs: 2, 4, 8-10, 15-18, and 34.

41. The expression cassette combination according to any of claims 1-40, wherein the second expression cassette comprises a nucleotide sequence set forth in any of SEQ ID NOs: 3, 5, 11-14, and 19-22.

42. The expression cassette combination according to any of claims 1-41, wherein the first expression cassette and the second expression cassette are present individually.

43. The expression cassette combination according to any of claims 1-42, wherein the first expression cassette and/or the second expression cassette are present in the form selected from the group consisting of an isolated nucleic acid molecule, a liposome, and/or an exosome.

44. The expression cassette combination according to any of claims 1-43, wherein the first expression cassette and/or the second expression cassette are present in the form of a plasmid.

45. The expression cassette combination according to any of claims 1-44, wherein the first expression cassette and/or the second expression cassette are viral vectors.

46. The expression cassette combination according to any of claims 1-45, wherein the first expression cassette and/or the second expression cassette are AAVs.

47. The expression cassette combination according to any of claims 1-46, wherein the molar ratio of the protein expressed by the first expression cassette to the protein expressed by the second expression cassette in the expression cassette combination is about 1:3 to 3:1.

48. The expression cassette combination according to any of claims 1-47, wherein the molar ratio of the protein expressed by the first expression cassette to the protein expressed by the second expression cassette in the expression cassette combination is about 1:1.

49. A kit comprising the expression cassette combination according to any of claims 1-48.

50. The kit according to claim 49, comprising reagents and/or instruments for administrating the expression cassette combination.

51. Use of the expression cassette combination according to any of claims 1-48 and/or the kit according to any of claims 49-50 in the manufacture of a medicament for treating a disease, wherein the disease comprises a disease caused by an ABCA4 mutation.

52. The use according to claim 51, wherein the disease comprises an inherited retinal disease.

53. The use according to any of claims 51-52, wherein the disease comprises a hereditary macular degeneration disease, age-related macular degeneration, retinitis pigmentosa, and/or cone-rod dystrophy.

54. A method for expressing a heterologous ABCA4 gene, comprising administrating the expression cassette combination according to any of claims 1-48 and/or the kit according to any of claims 49-50 to a subject in need thereof.

55. A method for alleviating the cell death caused by retinylphosphatidylethanolamine (NRPE), comprising administrating the expression cassette combination according to any of claims 1-48 and/or the kit according to any of claims 49-50 to a subject in need thereof.

56. A method for treating a disease caused by an ABCA4 mutation, comprising administrating the expression cassette combination according to any of claims 1-48 and/or the kit according to any of claims 49-50 to a subject in need thereof.

57. The method according to any of claims 54-56, wherein the administrating comprises injection.

58. The method according to any of claims 54-57, wherein the disease comprises an inherited retinal disease.

59. The method according to any of claims 54-58, wherein the disease comprises a hereditary macular degeneration disease, age-related macular degeneration, retinitis pigmentosa, and/or cone-rod dystrophy.
